# EUROPEAN PATENT SPECIFICATION

(11) **EP 3 225 245 B1**
(45) Date of publication and mention of the grant of the patent: **18.09.2019**
(21) Application number: 16382138.2
(22) Date of filing: 29.03.2016
(51) Int. Cl.: A61K 36/48, A61P 25/16

(54) **BIOACTIVE EXTRACT OBTAINED FROM VICIA FABA AND ITS USE IN THE TREATMENT AND/OR PREVENTION OF NEURODEGENERATIVE DISEASES**
VICIA FABA EXTRAKT UND SEINE VERWENDUNG ZUR BEHANDLUNG UND/ODER VORBEUGUNG NEURODEGENERATIVER ERKRANKUNGEN
EXTRAIT DE VICIA FABA ET SON UTILISATION DANS LE TRAITEMENT ET/OU PREVENTION DES MALADIES NEURODEGENERATIVES

(43) Date of publication of application: 04.10.2017
(73) Proprietor: Euroespes, S.A., 15165 Bergondo (A CORUÑA) (ES)
(72) Inventor: Cacabelos Garcia, Ramon, 15165 Bergondo (La Coruña) (ES)
(74) Representative: Pons

(56) References cited:
- US-A- 3 941 835
- KEMPSTER P A ET AL: "Motor effects of broad beans (Vicia faba) in Parkinson's disease: single dose studies", ASIA PACIFIC JOURNAL OF CLINICAL NUTRITION, SMITH-GORDON JOURNAL, LONDON, GB, vol. 2, no. 2, 1 June 1993 (1993-06-01), pages 85-89, XP009191250, ISSN: 0964-7058
- Rocco Longo ET AL: "DISTRIBUTION OF L-DOPA AND RELATED AMINO ACIDS IN VICIA", Phytochemstry, 1 January 1974 (1974-01-01), pages 167-171, XP055293483, Retrieved from the Internet: URL:http://ac.els-cdn.com/S003194220091287 1/1-s2.0-S0031942200912871-main.pdf?_tid=0 5c4c29c-5a1c-11e6-935d-00000aacb35e&acdnat =1470298888_1da677da20e6b20c384abb0042dc6e c6 [retrieved on 2016-08-04]

## Description

The present invention falls within the field of Neurosciences, specifically within bioactive extracts obtained by means of biotechnological processes from plant sources, which have pro-dopaminergic and neuroprotective activity, therefore being useful in the treatment and/or prevention of neurological pathologies associated with alterations in the dopaminergic system, particularly with a reduction in dopamine levels, such as Parkinson's disease or other Parkinsonian syndromes.

### STATE OF THE ART

Parkinson's disease (PD) is the most frequent disorder under the category of movement disorders, as well as the second neurodegenerative disease most prevalent in the population, only exceeded by Alzheimer's disease. It is estimated that 6.3 million people suffer from this disease in the world. Its incidence and prevalence increase with age, affecting 1%-2% of people aged 60 and over (average age of onset of the pathology) and increases to 4% in people aged 80 and over. These figures are estimated to triple in the next 50 years, due mainly to the ageing population.

PD mainly appears as a consequence of a slow and progressive degeneration of dopaminergic neurons in the *pars compacta* ventral area of the *substantia nigra* (black substance). This implies a reduction in nerve signals in the cortical-striatal-thalamic circuit, with the ensuing motor symptoms characteristic of this disease. The clinical manifestations begin when approximately three quarters of the dopaminergic neurons die in this region. Likewise, a neuronal loss normally occurs in other subcortical nuclei (Basal Nucleus of Meynert, *locus coeruleus,* raphe nucleus) and formation of Lewy bodies in the residual dopaminergic neurons. It has been postulated that this neurodegeneration could be related to oxidative stress mechanisms, excitotoxicity, apoptosis, mitochondrial dysfunction, inflammation and impairment of the ubiquitin-proteasome system.

The final cause of these neuropathological alterations is unknown, although at present the hypothesis that accumulates the greatest evidence is that which suggests an interaction of environmental and genetic factors. Both the early forms of onset and juvenile Parkinsonism are generally hereditary and due to genetic mutations. Other PA presentations are supported by the presence of certain genetic polymorphisms associated with a greater vulnerability to the disease (for example, the genes encoding α-synuclein, parkin or ubiquitin C-terminal hydrolase-L1, among others), in genes which have a mediating role in the pathological process or which are involved in multifaceted cascades and different metabolic reactions, as well as in the appearance of different diet-related environmental mechanisms, tobacco consumption, exposure to toxic substances, brain trauma, ischemic episodes, etc.

Although PD is a complex syndrome which affects a multitude of areas, it is the motor symptoms which are considered prototypical of this disease. The clinical diagnosis establishes the following manifestations as cardinal: resting tremor, rigidity, postural instability and bradykinesia and akinesia. In order to diagnose Parkinsonism, at least two of the cardinal symptoms must exist and, in addition, one of them must be tremor or rigidity. These manifestations usually commence unilaterally and have progressive and asymmetrical development, in which the greatest affectation coincides with the side on which the symptoms start and is usually aggravated by situations of stress and anxiety. In addition to the motor symptoms, patients with PD may have dysfunction of the autonomous nervous system, sensory disorders, cognitive impairment, neuropsychiatric alterations or sleep alterations.

In order to accurately characterise the clinical picture with regard to the pharmacological treatment, monitoring or research, it is necessary to apply psychometric tools that will make it possible to categorise and ascertain the severity of the cognitive and psychomotor impairment and its underlying functional repercussion and the possible associated psychological syndromes. This assessment is commonly supported by the administration of multidimensional scales that jointly quantify the patient's degree of impairment and provide information on their operation in the domains which are prototypically affected by this disease. These instruments include, namely: UPDRS - United Parkinson's Disease Rating Scales, ISAPD - Intermediate Scale for Assessment of Parkinson's Disease and SCOPA - Scale for Outcomes in Parkinson's Disease. However, especially in the case of doubtful or atypical cases in which differential diagnosis is crucial, it is important for the examiner to complement these batteries with specific scales that cover the altered functions in this disease more in depth (verbal fluency, visuospatial skills, executive functions, attention processes, memory, motor functions, etc.) or encompass a greater neuropsychological spectrum.

Although handling of PD requires complementary therapies focused on education, exercise and nutrition, pharmacological intervention is the preferred treatment for addressing this pathology and, according to the American Neurology Academy, it should be started once a significant impairment of functionality is detected in patients. The classical treatment of Parkinson's Disease consists of correcting dopamine neurotransmitter deficiency, deficient in the ganglions of the patient base with Parkinson's Disease and Parkinsonian syndromes, with the aim of alleviating the cardinal motor symptoms (Smith et al., 2012, Neuropsychopharmacology, 37: 213-246). In this regard, a replenishment of dopamine is sought through the administration of L-DOPA, it's precursor or active ingredients that increase the activity of that neurotransmitter on stimulating the dopaminergic receptors (dopaminergic agonists).

In 1910-1911, Torquato Torquati isolated L-dihydroxyphenylalanine (L-DOPA), the L-isomer of the amino acid D-dihydroxyphenylalanine, in the *Vicia faba* bean (Torquati T., 1913, Arch Farmacol sper, 15:213-223).

In 1913, Markus Guggenheim isolated the substance and defined its chemical structure. In 1938, Peter Holtz discovered L-DOPA decarboxylase, the enzyme that transforms L-DOPA into dopamine. In 1957, Kathleen Montagu and Weil-Malherbe and Bone demonstrated the presence of dopamine in different mammal tissues, including brain tissue. At the same time, Holtz demonstrated the stimulating effect of L-DOPA and postulated that dopamine was the active metabolite of L-DOPA in the brain. In 1957, Carlsson demonstrated that L-DOPA antagonised the calming effect of reserpine, the cause of a Parkinsonian syndrome in experimental animals. In 1957, Pletscher demonstrated that L-DOPA increased brain catecholamins levels, particularly dopamine. In 1959, the Bertler and Rosengren and Sano groups demonstrated that dopamine was located in the caudate nucleus and putamen, and postulated that dopamine could be involved in the Parkinsonian syndrome caused by reserpine. In 1960, Hornykiewicz demonstrated that dopamine deficiency in the caudate nucleus and putamen was responsible for PD. In 1967, Cotzias introduced the chronic treatment with L-DOPA as the standard treatment for Parkinson's Disease (Hornykiewicz, 2010, J. Neurol., Suppl. 2: S249-S252).

Previous studies with *Vicia faba* in PD disclosed the presence of L-DOPA in the bean of this leguminosa (Rabey et al., 1992, J. Neurol. Neurosurg. Psychiatry., 55(8): 725-727; Apaydin et al., 2000, Mov. Disord., 15(1): 164-166), although the processes developed to extract said molecule from this natural source have resulted in low yields, since the content of L-DOPA reached at the end of the process was approximately 5 mg/g.

Furthermore, there are other drugs that attempt to obtain the same effect by inhibiting enzymes that destroy dopamine, such as catechol-O-methyltransferase (COMT) and type B monoamino-oxidase (MAOB) (Chávez-Leon, Ontiveros-Uribe y Carrillo-Ruiz, 2013, Salud Ment., 36: 315-324).

Within this pharmacotherapeutical strategy, levodopa is the most effective treatment and which achieves the most significant improvement in the patient's functionality (Gazewood, Richards and Clebak, 2013, Am. Fam. Physicia., 87: 267-273); however, the maintenance of this treatment implies the appearance of motor fluctuations and dyskinesias within three to five years (or even earlier if the treatment is stated at an early stage or at excessively high doses). Dopaminergic agonists, while less likely to cause these effects, have significant adverse non-motor reactions (nausea and vomiting, hallucinations, confusion, drowsiness or sleep attacks, hypotension, impulse control disorders) (Fernandez, 2012, Cleve. Clin. J. Med., 79: 28-35). In the case of MAO-B inhibitor drugs, we find the opposite profile: it has fewer side effects but is also less effective. Also, the use of these drugs requires precaution with other drugs (such as amphetamines) and food. Furthermore, there is certain evidence that the use of COMT inhibitors such as tolcapone increases toxicity in the liver and that entacapone poses a greater cardiovascular and prostate cancer risk.

In addition to dopaminergic agents, anticholinergic drugs are used to relax smooth muscles, thereby reducing tremor, drooling and dystonia. However, they are scarcely effective with the other symptoms (rigidity, bradikynesia and postural instability), can alter the learning capacity and aggravate cognitive impairment, and have substantial side effects (constipation, urinary retention, dry eyes and dry mouth, blurred vision).

Therefore, the development of significant side effects, both motor and non-motor, in response to all types of currently available anti-Parkinsonian therapy, as well as the evidence that these drugs have not been proven to have a neuroprotective effect, evidences the need to seek new products that will make it possible to potentiate dopamine without implying serious harmful effects on other body systems and can therefore be used for long periods of time both at symptomatic and preventive level. Therefore, new therapeutic intervention methods are required in patients with brain injury in the dopaminergic system, which leads to the clinical expression of PD and/or different presentations of movement disorders whose common denominator is a Parkinsonian syndrome.

### DESCRIPTION OF THE INVENTION

The present invention provides a bioproduct or bioactive extract obtained from the *Vicia faba* pod, named E-PodoFavalin-15999 or Atremorine by its inventors, which has pro-dopaminergic, anti-inflammatory, antioxidant and neuroprotective activity, thereby improving the survival of dopaminergic neurons. Therefore, this bioproduct is proposed in this invention for the treatment and/or prevention of neurological disorders associated with a reduction in dopamine levels, such as Parkinson's Disease and other Parkinsonian syndromes of varying nature.

Therefore, the present invention represents a solution to the need for a new form of therapeutic intervention in patients with brain injury in the dopaminergic system, which gives rise to the clinical expression of Parkinson's Disease and/or different presentations of movement disorders whose common denominator is a Parkinsonian syndrome.

This bioproduct is obtained by means of a non-denaturing biotechnological procedure of the pod of different species of *Vicia faba* and, as shown in the examples of the present invention, has the following properties: (i) brain neurotrophic activity on different neuronal profiles; (ii) selective neuroprotective activity on dopaminergic neurons; (iii) neuronal antioxidant activity; (iv) neuronal anti-degenerative activity; (v) anti-degenerative and regenerative activity on animal models of Parkinson's Disease; (vi) psychomotor improvement (locomotion, coordination of movements, motor agility) in animal models of Parkinson's Disease; (vii) increase in dopamine synthesis (pro-dopaminergic activity) in more than 98% of Parkinson's patients or with Parkinsonian syndromes of varying nature (toxic, traumatic, degenerative, ischemic); (viii) hormonal regulation (hypothalamus-pituitary) dependent on dopaminergic neuromodulation; (ix) specific regulation of noradrenaline, prolactin, growth hormone and cortisol; and (x) personalised therapeutic effects in accordance with the pharmacogenetic profile of the patients. These properties are demonstrated in the present invention by means of three types of studies: (a) *in vitro* studies, (b) *in vivo* studies (animal models), and (c) clinical studies in Parkinson's patients and with various Parkinsonian syndromes. In addition, it should be noted that said bioproduct did not have adverse effects on the patients in the course of the *in vivo* assays.

Due to all these properties, the bioproduct E-PodoFavalin-15999 is indicated in pathologies such as: (i) Parkinson's Disease; (ii) Parkinsonian syndromes; (iii) extrapyramidal syndromes; (iv) diseases that cause dopamine or noradrenaline deficiency; (v) other neurodegenerative diseases (such as Alzheimer's Disease or Huntington's Disease); (vi) psychomotor disorders; (vii) diseases that cause increased prolactin (PRL) secretion; (viii) diseases that cause increased growth hormone (GH) production; (ix) stressful syndromes with increased cortisol production; and (x) any physiological and/or pathological condition that alters the production of dopamine, noradrenaline, PRL, GH or cortisol.

While previously written and published papers are focused on the chemical study of the bean of *Vicia faba,* the inventors of the present invention have developed a process based on the pod, whose properties had not been studied to date. This process leads to a bioproduct which has the improved properties described above and which also have the advantageous characteristic that it comprises at least 20 mg/g of L-DOPA, in comparison with the 5 mg/g obtained to date by means of other processes based on the bean. Thus, the high L-DOPA content of the *Vicia faba* pod prior to the maturation phase and its biomedical properties in animal models and in Parkinson's patients are shown for the first time. As it has been already mentioned, previous studies with *Vicia fava* in Parkinson's Disease relate to the properties of beans but do not consider the properties of the pod. Additionally, said previous studies are generally carried out on cooked beans, due to which the properties of the product are altered (Osman et al., 2014, J. Food Sci. Technol., 51(8): 1554-1560). In the case of the present invention, the entire preparation process of E-PodoFavalin-15999 is carried out under cold conditions, due to which the end product preserves all natural and biomedicinal properties of the *Vicia faba* pod, the exclusive substrate of E-PodoFavalin-15999.

As shown by the examples of the present invention, E-PodoFavalin-15999 is a powerful dopamine synthesis inducer due to the high L-DOPA content of the product (at least 20 mg/g), as opposed to beans, whose L-DOPA content is approximately 5 mg/g. Furthermore, the neurotrophic, antioxidant and anti-inflammatory effect of E-PodoFavalin-15999 is attributable to other primary and secondary metabolites present in this extract, such as carbohydrates, amino acids, organic acids, alkaloids, terpenoids, jasmonates and polyphenols. The *Vicia faba* pod also has a high flavonoid content and antioxidant activity. Other components of E-PodoFavalin-15999 with biomedicinal properties could be abscisic acid or betalains, water-soluble pigments whose main structural component, betalamic acid, is formed from the amino acid 4,5-dihydroxyphenylalanine by the action of the 4,5-DOPA-extradiol-dioxygenase enzyme. Vicine [2,6-diamino-4,5-dihydroxypyrimidine-5-(β-D-glucopyranoside)] and convicine [2,4,5-trihydroxy-6-aminopyridina-5-(β-D-glucopyranoside)] are phenolic compounds of condensed tannins which are present in *Vicia faba,* acting as anti-nutritional compounds. The aglycones (divicine and isourramyl) of these two pyrimidine glycosides are the agents responsible for favism, a genetic disease that causes hemolytic anemia in males with erythrocyte glucose-6-phosphate-dehydrogenase deficiency (EC.1.1.49, G6PD). E-PodoFavalin-15999 contains residual levels of vicine and convicine, below 1-2 mg/g. Therefore, another advantage of the bioproduct of the invention is that the absence of vicine and convicine therein eliminates all risk of favism in people who consume this product, even if they have G6PD deficiency.

Due to the foregoing, the present invention represents an enhancement with respect to the therapeutic strategies used to date to treat Parkinson's Disease, particularly compared to other similar bioproducts obtained from the *Vicia faba* bean which also contain L-DOPA.

Therefore, a first aspect of the present invention relates to a process, hereinafter "process of the invention", for obtaining a bioactive extract from the *Vicia faba* pod that comprises the following stages:
a. harvesting the fruit of a *Vicia faba* crop during the growth phase,
b. removing the beans from the fruit harvested in stage (a) and processing the pod by means of a treatment that comprises the steps of hygienisation, aseptisation and removal of contaminating elements,
c. preserving the product obtained in stage (b) at a temperature between 0°C and 5°C for 24-48 hours,
d. deep-freezing the product obtained after stage (c) at between - 50°C and -30°C, preferably at -40°C, for 1-3 hours,
e. storing the product obtained after stage (d) at a temperature below -18°C for less than three months,
f. freeze-drying the product obtained after stage (e), preferably for 3-5 days, and
g. standardising the L-DOPA content of the product obtained after stage (f) such that it has a final L-DOPA content of at least 20 mg/g.

The crop mentioned in stage (a) of the process of the present invention is sown, preferably, in spring-summer and the fruit is harvested, more preferably, in December-January. "Growth phase" is understood to be any stage of the crop prior to commencing the maturation phase. Persons skilled in the art will recognise said crop development phases. Preferably, the harvest takes place when the pods are still green and before the skin of the seeds begins to roughen. In a more preferred embodiment of the process of the invention, in stage (a) the fruit is harvested two to three weeks before the crop enters the maturation phase. This harvesting stage is a key stage for the subsequent obtainment of the final bioactive extract or product (E-Podo-Favalin-15999), both for standardising L-DOPA levels and for stabilising the therapeutic effects of the final bioproduct.

The common or vulgar name of *Vicia faba* is broad bean. It belongs to the Kingdom: Plantae, Division: Magnoliaphyta, Class: Magnoliopsida, Subclass: Rosidae, Order: Fabales, Family: Fabaceae, Subfamily: Faboideae, Tribe: Fabeae, Genus *Vicia,* Species: *V. faba.* Its Latin name is *Vicia faba* (synonyms: *Faba bona Medik., Faba equina Medik., Faba faba (L.) Housa, Faba major Desf., Faba minor Roxb., Faba sativa Bernh., Faba vulgaris Moench, Orobus faba Brot., Vicia esculenta Salisb., Vicia vulgaris Gray).* It belongs to the Leguminosae family.

The "fruit" is a legume having an elongated pod of varying length, preferably between 10 cm and 30 cm and with a fleshy consistency, with a spongy partition with a kind of velvety hair or false partition between the seeds, which are more or less flat. Inside this pod there are seeds or beans arranged in a row. The pod, which is green when immature, darkens and becomes pubescent when dry. The seeds in the interior of the pod vary preferably between two and nine. The seeds are oblong, more or less large in size, depending on the variety, and yellowish-green which, when over-maturing, turns bronze. There are also blackish and purple seed varieties. The seeds preferably weigh 1-2 g. The germination capacity lasts preferably four to six years. In commercial seeds, the minimum germination percentage is 90% and the minimum purity is 99%. Therefore, in the present invention "fruit" is understood to be the legume of *Vicia faba,* which comprises a pod and seeds or beans in its interior, and "bean" is understood to be each of the seeds that the *Vicia faba* pod contains in its interior.

The *Vicia faba* plant is straight and erect with strong, angular stems preferably up to 1.6 m in height. It has alternate leaves, paripinnate and compound, with broad, dark green, oval-rounded leaflets without tendrils; the terminal leaflet does not exist or becomes a rudimentary tendril. The flowers are arranged in racemes of preferably two to eight axillary flowers, which are fragrant and large, reaching preferably 4 cm, with white petals tinged with violet, purple or black. They are hermaphrodites and the plant is capable of self-pollination. The root of *Vicia faba* grows deep until reaching a length similar to that of the stem of the plant. Like other fabaceae, its nodules have the property of fixing nitrogen to the soil; although up to 80% thereof is consumed by the plant itself, the remaining 20% enhances soil fertility.

In another preferred embodiment of the process of the invention, the *Vicia faba* crop is an organic crop. In the present invention, "organic crop" is understood to be a crop in which no chemical product is used to treat the plants or soil, such as pesticides or herbicides.

As regards the preferred cultivation conditions of *Vicia faba,* although this plant is not among the most demanding, it prefers temperate to warm uniform temperatures and oceanic climates are preferred over continental climates. The optimum temperature is preferably 15°C to 22°C. As regards the type of soil, it can be grown in any well-drained soil. It is not very demanding, although clayey or siliceous soils and calcareous-clay soils rich in humus, deep and cool, are preferred. It does not grow well in poorly drained wet soils. Optimum pH ranges preferably between 7.3 and 8.2. In cold climates it is preferably sown in spring. The sowing season is linked to the climate and is carried out from August-September in early crops until November and in inland areas it is sown in spring. It is preferably sown in a steady flow, strokes, by hand or using a seed drill. The seeds are preferably arranged in lines or ridges with a distance between lines of preferably between 50-60 cm and 25-30 cm between plants. Emergence takes place 8-12 days later, depending on the temperature. The largest seeds are sown preferably at a depth of 5 cm and preferably at a distance of 25 cm therebetween, in well stirred and fertilised soil.

There are the following varieties of green beans: Aguadulce (Seville) (semi-early maturing, violet stems, large elongated pods, toasted cream-coloured seeds), Muchamiel (very early-maturing, medium-sized plant, reddish stems, hanging pods, toasted cream-coloured seeds), Reina Blanca (less early-maturing than Muchamiel, grayish white-coloured seeds), Granadina (light-coloured seeds), Reina Mora (purple seeds), Arbo (also called Blanca, erect, white-coloured seeds, green stems), Mahón (two modalities: white and purple; the white modality has reddish-coloured seeds and the purple modality has violet-coloured seeds; medium-sized, semi-erect). Depending on the size of its seeds or beans, *Vicia faba* is classified into three varieties: *Vicia faba* var. *minor,* whose seeds are small, weighing between 0.3 g and 0.7 g each, ellipsoidal, with a cylindrical pod which can be up to 15 cm long; *Vicia faba* var. *Equina,* whose seeds are medium-sized and flat, weighing between 0.7 g and 1.1 g, with moderately deshicent pods; *Vicia faba* var. *major,* the most frequently used for fresh consumption, the seeds weigh between 1.2 g and 1.8 g, with indehiscent pods which can be up to 35 cm long. The most widespread crop, that called Aguadulce, belongs to this variety.

In another preferred embodiment of the process of the invention, the cultivation of *Vicia faba* to which reference is made in stage (a) is of the Muchamiel variety, i.e. *Vicia faba L.* (sp. var. MM). Prior to step (a) of the process of the present invention, an optional step in which the different strains of *Vicia faba* are genetically classified and characterised can be carried out, with the aim of subsequently selecting the variant, preferably the variant Muchamiel (MM), for cultivation with maximum germination performance.

In step (b) of the process of the invention all the components of the bean are removed. E-PodoFavalin-15999 is a bioproduct derived from the components of the *Vicia faba* pod. Therefore, all the components of the bean are removed in this extraction stage. This selective treatment of the pod is essential for preserving the L-DOPA content and the other natural components that integrate the final organic structure of E-Podo-Favalin-15999. Step (b) therefore comprises a selective pod treatment process which comprises the steps of hygienisation, aseptisation and removal of contaminating elements. "Hygienisation" is the washing of the pod, preferably a quick wash, more preferably by means of a chlorinated water jet. "Aseptisation" is an optimal product (pod) selection process that rejects damaged and/or oxidised products. The last step in the "removal of contaminating elements" comprises the removal of residue, preferably through the washing and subsequent quick-drying of the raw material (pod) selected.

During step (c) of the process of the invention, the product is preserved, preferably at 0°C, for 24-48 hours. The entire extraction process of E-Podo-Favalin-15999 is carried out under cold conditions. Temperatures above 5°C denaturalise the product and eliminate its properties.

During step (d) of deep-freezing, the product is deep-frozen at, preferably, -40°C for between one and three hours. This is an essential step for stabilising possible enzymatic mechanisms that denaturalise the product.

During step (e) of product storage, the product is stored at, preferably, - 20°C for a period of less than three months. Until the stage subsequent to freeze-drying, the product must be kept at temperatures below -18°C.

Stage (f) of industrial freeze-drying of the product is carried out preferably between three and five days. This freeze-drying process comprises three phases: (1) Deep-Freezing Phase at preferably -80°C for preferably 12 hours to generate a frozen matrix rich in crystalline structures that facilitate the sublimation process; (2) Sublimation Phase or primary vacuum desiccation preferably between 24 and 48 hours, until reducing the water content of the product at a level of preferably between 3%-5%; and (3) Desorption Phase or secondary desiccation at a controlled temperature for preferably three to five days, until the water content in the interior of the product is approximately 0 (<0.5%).

Next, the product obtained after stage (f) is subject to a post-freeze-drying non-denaturalising treatment process. This post-freeze-drying treatment comprises an optional step of analysing the chemical composition of the product and a L-DOPA content standardisation step. This "standardisation" step is carried out by means of the homogenisation of lots until reaching an adequate average L-DOPA content. After this standardisation step, the product must have a final content of L-DOPA of at least 20 mg/g, preferably 25 mg/g.

In a more preferred embodiment, the process of the invention also comprises a stage (h) of homogenisation of the product obtained after stage (g), which comprises standardisation of the texture and appearance of the final product by means of a neutral physical treatment. This "neutral physical treatment" preferably comprises grinding and filtering steps.

In an even more preferred embodiment of the process of the invention, it also comprises a stage (i) of formulation wherein between 0.020 g and 0.040 g of D-Alpha-Tocopherol are added. In this stage, preferably, 0.033 g of D-Alpha-Tocopherol are added, equivalent to 12 mg of vitamin E (Tocopherol) (100% RDI).

The final result of the process of the invention is a bioactive extract or bioproduct, called "E-PodoFavalin-15999" or "Atremorine" in the present invention, which is preferably presented in the form of a homogeneous greenish-brown powder and which can be included in four formats: (i) 250 mg capsules; (ii) 5 g bags; (iii) 75 g bottles; and (iv) 150 g bottles.

This final product, in its different presentations, contains a concentration of L-DOPA of at least 20 mg/g and vicine and convicine levels of less than 1-2 mg/g.

Another aspect of the invention relates to a bioactive extract, hereinafter "bioactive extract of the invention", "extract of the invention", "bioproduct of the invention", "Atremorine" or "E-PodoFavalin-15999", obtained or obtainable by means of the process of the invention. Preferably, said extract of the invention is in powdered form.

In a preferred embodiment, the bioactive extract of the invention comprises an amount of L-DOPA of at least 20 mg/mg and amounts of vicine and convicine of less than 2 mg/g, preferably less than 1 mg/g.

In another preferred embodiment, the bioactive extract of the invention comprises the following composition:
a. Between 60% and 70%, preferably 65%, of carbohydrates,
b. Between 10% and 20%, preferably 17%, of proteins,
c. Between 0.5% and 1 %, preferably 0.7%, of fat,
d. Between 15% and 25%, preferably 20%, of dietary fibre,
e. Less than 3ppm of gluten,
f. Between 5% and 10%, preferably 9%, of water,
g. Between 3% and 8%, preferably 6%, of ashes,
h. Between 400 and 500 mg/100g, preferably 440 mg/100 g, of calcium,
i. Between 5 and 15 mg/100g, preferably 10 mg/100 g, of iron,
j. Between 200 mg/100 g and 250 mg/100 g, preferably 205 mg/100 g, of magnesium,
k. Between 1mg/100g and 3mg/100g, preferably 2 mg/100 g, of manganese,
l. Between 1500 and 2000 mg/100 g, preferably 1,800 mg/100 g, of potassium,
m. Between 300 and 400 mg/100g, preferably 385 mg/100 g, of sodium,
n. Between 10% and 20%, preferably 15%, of amino acids:
   Aspartic: between 3% and 4%, preferably 3.52%
   Glutamic: between 2% and 3%, preferably 2.30%
   Arginine: between 1% and 2%, preferably 1.20%
   Leucine: between 0.2% and 1%, preferably 0.96%
   Isoleucine: between 0.2% and 1%, preferably 0.42%
   Lysine: between 0.2% and 1%, preferably 0.87%
   Tyrosine: between 0.2% and 1%, preferably 0.86%
   Threonine: between 0.2% and 1%, preferably 0.86%
   Serine: between 0.2% and 1%, preferably 0.83%
   Alanine: between 0.2% and 1%, preferably 0.73%
   Proline: between 0.2% and 1%, preferably 0.70%
   Glycine: between 0.2% and 1%, preferably 0.61%
   Phenylalanine: between 0.2% and 1%, preferably 0.57%
   Valine: between 0.2% and 1%, preferably 0.37%
   Histidine: between 0.2% and 1%, preferably 0.31%
o. At least 20 mg/g, preferably 25 mg/g, of L-DOPA,
p. Between 2 and 3 mg/g, preferably 2.4 mg/g, of vitamin E,
q. Less than 1% de vicine,
r. Less than 1% of convicine,
s. Less than 1% of lecitine,
t. Less than 1% of coline.

The energetic value of the bioactive extract of the invention is, preferably, 340 Kcal/100 g and 1,440 Kjul/100 g.

Another aspect of the invention relates to a composition which comprises the aforementioned elements (a) to (t).

Another aspect of the invention relates to a pharmaceutical composition, hereinafter "pharmaceutical composition of the invention" or "composition of the invention", which comprises the bioactive extract of the invention.

The composition of the invention comprises the bioproduct of the invention in a therapeutically effective amount. "Therapeutically effective amount" is understood to be the amount of extract of the invention that, when administered to the patient to treat and/or prevent a neurological condition based on a reduction in dopamine levels, produces the desired effect, thereby increasing said levels. The therapeutically effective amount may vary depending on a variety of factors, for example
the type of neurological condition or impairment and its severity, as well as age, weight, sex, physical condition, response or tolerance capacity, etc., of the individual to whom the composition of the invention is going to be administered.

As shown in the examples shown below, the administration of 5 g of the bioproduct of the invention to Parkinson's patients induced a spectacular serum dopamine response. Therefore, the bioactive extract is preferably found in an amount of between 3 g and 7 g, more preferably 5 g, in the composition of the invention.

In a more preferred embodiment, the composition of the invention also comprises a pharmaceutically acceptable vehicle and/or other active ingredient. As shown in the examples below, the co-administration of the bioproduct of the invention, together with other L-DOPA-based anti-Parkinsonian drugs potentiates the therapeutic effect of the latter on potentiating the increase in dopamine levels caused by these. Therefore, the other active ingredient is preferably an anti-Parkinsonian drug, more preferably L-DOPA and, even more preferably, Sinemet.

"Anti-Parkinsonian drug" is understood to be any drug used to treat or prevent Parkinson's Disease or other Parkinsonian syndromes. Examples of these types of drugs are L-DOPA, dopaminergic agonists, COMT or MAO-B inhibitors, levodopa, anticholinergic drugs, etc.

The pharmaceutically acceptable excipients and vehicles that may be used in the composition of the invention are those known by the persons skilled in the art.

The term "excipient" makes reference to a substance which aids the absorption of the elements of the composition of the invention, stabilises said elements, activates or helps to prepare the composition in the sense of giving it consistency. Therefore, excipients may have a bonding function for keeping the ingredients bonded together, such as for example in the case of starches, sugars or celluloses, a sweetening function, a dyeing function, a protective function for protecting the composition, such as for example to isolate it from the air and/or humidity, a filling function for filling a pill, capsule or any other form of presentation, such as for example in the case of dibasic calcium phosphate, a disintegrating function to facilitate the dissolution of the components and their absorption, not excluding any type of excipients not mentioned in this paragraph.

The "pharmaceutically acceptable vehicle", like the excipient, is a substance or combination of substances used in the composition to dilute any of the components comprised therein up to a certain volume or weight. The term "vehicle" refers to a solvent, coadjuvant, excipient or carrier with which the composition of the invention must be administered; obviously, said vehicle must be compatible with said composition. Pharmaceutically acceptable vehicles may be solids, liquids, solvents or surfactants. Examples of vehicles may be water, oils or surfactants, including those of petroleum, animal, vegetable or synthetic origin, such as for example peanut oil, soybean oil, mineral oil, sesame seed oil, castor oil, polysorbates, sorbitan esters, ether sulfates, sulfates, betains, glucosides, maltosides, fatty alcohols, nonoxinoles, poloxamers, polioxiethylenes, poliethylenglycols, dextrose, glycerol, digitonin and similar. The pharmacologically acceptable vehicle is an inert substance or vehicle having an action similar to any of the elements comprised in the composition of the present invention. The function of the vehicle is to facilitate the incorporation of other elements, enable better dosing and administration or give consistency and format to the composition. When the format of the presentation is liquid, the pharmacologically acceptable vehicle is the solvent.

The composition of the invention and/or its formulations may be administered in a variety of ways including parenteral, intraperitoneal, intravenous, intradermal, epidural, intraspinal, intrastromal, intraarticular, intrasinovial, intratecal, intralesional, intraarterial, intracardiac, intramuscular, intranasal, intracranial, cutaneous or subcutaneous, oral, intraorbital intracapsular, topic, ophthalmological or ocular, by means of transdermal patches, percutaneous, nasal spray, surgical implant, internal surgical paint, infusion pump or via catheter.

The compositions of the present invention are also suitable for being applied by means of medical devices which make it possible to release the active ingredient(s) in adequate concentrations for treating and/or preventing neurological conditions based on a reduction in dopamine levels, such as for example Parkinson's Disease or Parkinsonian syndromes. These devices must be, preferably, appropriate for locally administering the active ingredient(s), allowing the treatment to act on the affected region and not be dispersed. The devices can, for example include the active ingredient(s) in their interior or be coated therewith.

In another preferred embodiment, the pharmaceutical composition of the invention is formulated for the oral administration thereof.

The composition of the present invention can be formulated for administration to an animal, preferably a mammal, including humans, in a variety of ways known in the state of the art. Examples of preparations could include any solid composition (tablets, pills, capsules, bags, bottles, powders, granules, bars, pencils, vaporisers, aerosols, etc.), semi-solid (ointment, cream, balm, gel, hydrogel, foam, lotion, soap, gelatin, etc.) or liquid (aqueous or nonaqueous solutions, hydroalcoholic or hydroglycolic solutions, suspensions, emulsions, syrups, anhydrous compositions, aqueous dispersions, oils, milks, balsams, liniments, serums, etc.) for oral, topic or parental administration, preferably oral. The composition of the present invention may also be in the form of sustained release formulations or any other conventional release system. The term "sustained release" is used in the conventional sense in reference to a compound vehiculisation system that enables the gradual release of said compound during a period of time and preferably, although not necessarily, with relatively constant compound release levels over a period of time. Illustrative examples of sustained release vehicles or systems include liposomes, mixed liposomes, oleosomes, niosomes, etosomes, milicapsules, microcapsules, nanocapsules, sponges, cyclodextrins, blisters, micelles, mixed surfactant micelles, mixed surfactant phospholipid micelles, milispheres, microspheres, nanospheres, lipospheres, microemulsions, nanoemulsions, miniparticles, miliparticles, microparticles, nanoparticles, solid lipid nanoparticles, nanostructured lipid media, polymer materials, biodegradable or non-biodegradable patches or implants, or biodegradable microparticles, such as for example biodegradable microspheres.

In another preferred embodiment, the pharmaceutical composition of the invention is included in a capsule, bag or bottle.

The examples shown below demonstrate that the bioactive extract of the invention has a neuroprotective effect on a human neuroblastoma cell line, on rat hippocampus in an oxygen and glucose deprivation model and on rat striatum in an induced toxicity model. Likewise, said extract avoids the neurodegeneration of dopaminergic nigrostriatal neurons in murine models of Parkinson's Disease, increasing neuronal survival, and both in animal models and in Parkinson's patients the bioproduct induced a surprising increase in dopamine levels and enhanced motor and learning functions. Therefore, the extract of the invention has neuroprotective and anti-inflammatory effects against the degenerative effects of Parkinson's Disease, due to which it has therapeutic value in the treatment of the neurodegenerative aspects of Parkinson's Disease relating to dementia and cognitive impairment

The bioactive extract of the invention also induced an increase in noradrenaline levels and a reduction in prolactin, cortisol and growth hormone levels in Parkinson's patients.

Therefore, another aspect of the invention relates to the bioactive extract of the invention or to the pharmaceutical composition of the invention for use as a medicament. Alternatively, this aspect of the invention relates to the use of the bioactive extract of the invention or to the composition of the invention for the manufacture of a medicament.

Another aspect of the invention relates to the bioactive extract of the invention or to the pharmaceutical composition of the invention for use in the treatment and/or prevention of neurodegenerative diseases, preferably Parkinson's Disease or other Parkinsonian syndromes.

"Neurodegenerative diseases" is understood to be a type of diseases that groups together a class of cognitive disorders, such as for example Alzheimer's Disease, Parkinson's Disease, Creutzfeldt-Jakob Disease, Huntington's Disease, Friedreich's ataxia, dementia with Lewy bodies, spinal muscular atrophy, amyotrophic lateral sclerosis and multiple sclerosis. These cognitive disorders are due to an increase in cell death processes, reducing the number of neurons and giving rise to changes in behaviour. Neurodegenerative diseases affect various activities such as balance, movement, speech, breathing and heart functions. Many of these diseases are genetic, but in the present invention both genetic and those derived from other different causes are included. The causes can be alcoholism, smoking, tumours or cerebrovascular accident (CVA). Other causes include toxins, chemicals and viruses.

"Parkinsonian syndromes" are understood to be syndromes of varying nature, for example toxic, traumatic, degenerative or ischemic syndromes that cause neurodegeneration and a cognitive and locomotive impairment similar to those of Parkinson's Disease and which also cause a reduction in dopamine levels. They are caused by disorders in the extrapyramidal system, which is a set of nerve pathways and centres located outside and independent of the pyramidal or corticospinal system, which intervenes in the regulation of involuntary motility. Parkinsonian syndromes are a neurological disorder whose symptoms are similar to those found in Parkinson's Disease. They may be due to an authentic secondary Parkinson's Disease or to the degeneration of certain neurons in the brain that cause a deficiency in the neurotransmitter dopamine. Other causes are possible, such as an abnormally high amount of copper in the body (Wilson's Disease), the ingestion of neuroleptic drugs or other degenerative diseases of the nervous system. Parkinsonian syndrome causes different symptoms, such as akinesia, i.e. slower and scarce movement, tremors at rest and muscular rigidity.

The medical uses proposed herein for the bioactive extract of the invention and for the pharmaceutical composition of the invention are extensive to the treatment and/or prevention of the diseases or pathologies selected from the list consisting of: brain injury associated with oxygen and/or glucose deprivation, brain injury associated with toxicity induced by external agents, neuroblastoma, extrapyramidal syndromes, diseases that cause dopamine or noradrenalin deficiency, other neurodegenerative diseases (such as for example Alzheimer's Disease or Huntington's Disease), psychomotor disorders, diseases that cause increased prolactin (PRL) secretion, diseases that cause increased production of growth hormone (GH), stressful syndromes with increased production of cortisol and any physiological and/or pathological condition that alters the production of dopamine, noradrenalin, PRL, GH or cortisol.

As shown by the examples of the present invention, the therapeutic effects of the bioproduct of the invention are particularly potentiated in the subgroup of Parkinson's patients with the APOE -2/3 genotype and/or the CYP2D6-IM phenotype. Therefore, a more preferred embodiment relates to the bioactive extract of the invention or to the pharmaceutical composition of the invention for use in the treatment and/or prevention of Parkinson's Disease, even more preferably in patients with the genotype APOE -2/3 and/or the phenotype CYP2D6-IM, wherein the phenotype CYP2D6-IM is a pharmacogenetic profile corresponding to patients classified as intermediate metabolisers.

The apolipoprotein E (APOE) gene, located in 19q 13.2, has three alleles in humans (ε2, ε3, ε4) which give rise to four prevalent genotypes: APOE-2/2 (<1%), APOE-2/3 (2-5%), APOE-2/4 (1-2%), APOE-3/3 (50-60%), APOE-3/4 (20-30%) and APOE-4/4 (1-3%). The genotyping of the APOE gene (APOE rs429358 c.3932T>C; Cys112Arg; C_3084793_20) is performed by means of conventional methods known in the art for analysing DNA extracted preferably from whole blood, more preferably by means of venipuncture.

The CYP2D6 gene belongs to the P450 Cytochrome family and encodes one of the most important enzymes for metabolising the drugs. More than 60% of the drugs that act on the nervous system are metabolised through the pathway CYP2D6. The CYP2D6 gene has more than 100 allelic variants in the human population, which configure four prevalent phenotypes: (i) normal metabolisers (Extensive Metabolisers) (EM), with the two normal alleles and full enzymatic activity; (ii) intermediate metabolisers (Intermediate Metabolisers) (IM), with a mutant allele and loss of 25%-50% of its enzymatic capacity; (iii) poor metabolisers (Poor Metabolisers) (PM), with two mutant alleles and no enzymatic capacity; and (iv) ultra-rapid metabolisers (Ultra-Rapid Metabolisers) (UM), with multiplied functional alleles and magnified enzymatic capacity.

The term "drug", as used in this invention, makes reference to any substance used to prevent, alleviate, treat or cure the diseases or pathologies described previously in this document, in humans, or in any other animal. In the context of the present invention, this term relates to a preparation that comprises the bioactive extract of the invention.

The drug to which the present invention refers may be for human or veterinary use. The "drug for human use" is any substance or combination of substances presented as having properties for treating or preventing diseases in human beings or that can be used in human beings or administered to humans for the purpose of restoring, correcting or modifying physiological functions by exercising a pharmacological, immunological or metabolic action or for establishing a medical diagnosis. The "drug for veterinary use" is any substance or combination of substances having curative or preventive properties with respect to animal diseases or that can be administered to the animal in order to restore, correct or modify its physiological functions by exercising a pharmacological, immunological or metabolic action or establishing a veterinary diagnosis including medicated premixes. "Medicated premix" or "medicated food premix" is understood to be any veterinary drug previously prepared with a view to the subsequent manufacture of medicated foods. "Medicated food" is understood to be any mixture of veterinary drug(s) and food(s) prepared prior to the marketing thereof and destined for being administered to animals without transformation, in respect of the curative or preventive or other properties of the drug.

The drugs of the invention may be used both alone and in combination with other drugs or compositions for treating or preventing neurodegenerative diseases or any of the other pathologies described above, preferably Parkinson's Disease or other Parkinsonian syndromes, more preferably with other drugs such as L-DOPA. Thus, the drugs of the present invention may be used together with other active ingredients or therapies in the manner of a combined therapy. The other active ingredients may form part of the same composition or can be provided by means of a different composition, being administered at the same time or at different times (simultaneous or sequential administration).

The term "treatment", as understood in the present invention, refers to combating the effects caused as a result of the disease or pathological condition of interest in an individual (preferably a mammal and, more preferably, a human) which includes:
(i) inhibiting the disease or pathological condition, i.e. interrupting its course;
(ii) alleviating the disease or pathological condition, i.e. causing the regression of the disease or pathological condition or its symptoms;
(iii) stabilising the disease or pathological condition.

The term "prevention", as understood in the present invention, consists of avoiding the appearance of the disease or pathological condition in an individual (preferably a mammal and, more preferably, a human), particularly when said individual is susceptible of developing the disease or pathological condition but has not yet been diagnosed, as in the case, for example, of family members of patients with a neurodegenerative disease, preferably Parkinson's Disease or other Parkinsonian syndromes.

Throughout the description and claims, the word "comprises" and its variants are not intended to exclude other technical characteristics, additives, components or steps. For the persons skilled in the art, other objects, advantages and characteristics of the invention will be partly inferred from the description and partly from the practice of the invention. The following examples and figures are provided by way of illustration of the present invention.

### DESCRIPTION OF THE DRAWINGS

**Figure 1** shows the protective effect of Atremorine in a SH-SY5Y human neuroblastoma cell line culture.
**Figure 2** shows the protective effect of Atremorine in the oxygen and glucose deprivation (OGD) model in slices of rat hippocampus.
**Figure 3** shows the protective effect of Atremorine in the 6-OHDA toxicity model in slices of rat striatum.
**Figure 4** shows the effect of Atremorine on dopaminergic degeneration in a MPTP murine model.
**Figure 5** shows the effect of Atremorine on microglia in a MPTP murine model.
**Figure 6** shows the effect of Atremorine on pshychomotor activity in a MPTP murine model analysed in Rota-Rod. Psychomotor activity level (counts).
**Figure 7** shows the effect of Atremorine on psychomotor activity and cognitive functions in a MPTP murine model analysed in the OUCEM-86 (Osaka University Computerised Electronic Maze). Psychomotor/psychomotor efficiency activity level (it can be seen that Atremorine (4 mg) is capable of reversing the toxic effect of MPTP, restoring the normal psychomotor function in the treated animals).
**Figure 8** shows the effect of Atremorine on serum dopamine levels in previously untreated Parkinson's patients.
**Figure 9** shows the effect of Atremorine on serum dopamine levels in previously untreated Parkinson's patients (left), pretreated patients (Sinemet, centre) and in the total group (patients pretreated with Sinemet + previously untreated patients, right).
**Figure 10** shows the effect of Atremorine on serum dopamine levels in previously untreated Parkinson's patients based on the APOE genotype.
**Figure 11** shows the effect of Atremorine on serum dopamine levels in previously untreated Parkinson's patients based on the CYP2D6 geno-phenotype (EM: Extensive Metabolisers; IM: Intermediate Metabolisers; PM: Poor Metabolisers; UM: Ultra-Rapid Metabolisers).
**Figure 12** shows the effect of Atremorine on serum noradrenaline levels in previously untreated Parkinson's patients (left), pretreated patients (Sinemet, centre) and in the total group (patients pretreated with Sinemet + previously untreated patients, right).
**Figure 13** shows the effect of Atremorine on serum PRL levels in previously untreated Parkinson's patients (left), pretreated patients (Sinemet, centre) and in the total group (patients pretreated with Sinemet + previously untreated patients, right).
**Figure 14** shows the effect of Atremorine on serum cortisol levels in previously untreated Parkinson's patients (left), pretreated patients (Sinemet, centre) and in the total group (patients pretreated with Sinemet + previously untreated patients, right).
**Figure 15** shows the effect of Atremorine on serum GH levels in previously untreated Parkinson's patients (left), pretreated patients (Sinemet, centre) and in the total group (patients pretreated with Sinemet + previously untreated patients, right).

### EXAMPLES

Following is an illustration of the invention by means of assays conducted by the inventors which demonstrate the effectiveness of the bioactive extract of the invention in the treatment of disorders associated with a reduction in dopamine levels, for example in Parkinson's Disease.

### Example 1. In vitro studies.

### 1.1 Evaluation of the protective effect of Atremorine in a SH-SY5Y human neuroblastoma cell line culture.

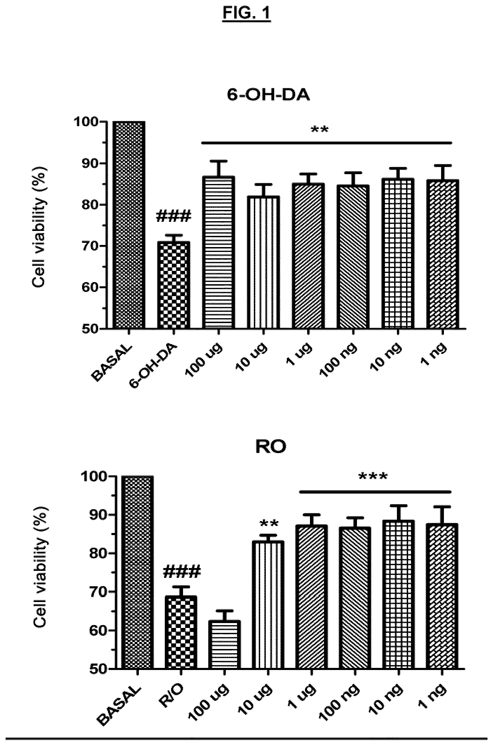

The SH-SY5Y cells were kept in a culture medium with 10% of inactivated fetal bovine serum, 15 non-essential amino acids, 1 mM of sodium pyruvate (Invitrogen, Madrid, Spain), F12 nutrient medium (F12 Ham), MEM medium (Eagle's Minimum Essential Medium) (Sigma Aldrich, Madrid, Spain), NaHCO₃, 100 u/ml Penicillin and 100 µg/ml of streptomycin (Invitrogen, Madrid, Spain) in H₂O miliQ. The cells were initially cultivated in a bottle and subcultures were subsequently prepared on 48-well plates with a density of 1x10⁵ cells/well. The cells were kept in an incubator at 37°C in a humidified 5% CO₂ atmosphere. Before reaching confluence, the cells were incubated with Atremorine at 1, 10 and 100 ng/ml; and at 1, 10 and 100 µg/ml for 24 hours; after that period had elapsed, the cells were incubated with two toxic stimuli, 6-Hydroxydopamine (6-OHDA) and a 30 µM Rotenon/10 µM Oligomycin-A (RO) cocktail in the presence of Atremorine for evaluating its therapeutic potential and determining the concentration that induces neuroprotection for undertaking subsequent assays. The results are presented in figure 1.

As can be observed in figure 1, 24 hours of treatment with 100 µM 6-OHDA produced 30% cell death; in these conditions, the incubation of Atremorine at all the assayed concentrations (1 ng/ml-100 µg/ml) produced a significant protective effect of, approximately, 50%. Secondly, the potential protective effect of Atremorine was evaluated in an oxidative stress and mitochondrial dysfunction model produced by the combination of Rotenon and Oligomycin-A (Fig. 1). The exposure of the cells to Rot/Oligo for 24 hours produced 32% cell death; as in the previous case, incubation with Atremorine produced a 60% protective effect at concentrations of 1ng/ml- 10 µg/ml. However, no protection was observed at the highest concentration (100 µg/ml).

### 1.2 Evaluation of the protective effect of Atremorine in the oxygen and glucose deprivation (OGD) model on slices of rat hippocampus.

These experiments are conducted on Sprague-Dawley rats from a colony at the animal facility. The rats are anaesthetised using sodium barbital (69 mg/kg, i.p.). Next, they are decapitated and the brain removed and quickly immersed in a pre-cooled bicarbonate Krebs solution (with high magnesium and sacarose and low calcium content) and whose composition, in mM, is as follows: NaCl 120, KCI 2, CaCl₂ 0,5, NaHCO₃ 26, MgSO₄ 10, KH₂PO₄ 1.18, glucose 11 and sacarose 200. All the solutions are previously bubbled with 95% O₂/5% CO₂ (carbogen) or 95% N₂/5% CO₂ (nitrogen) to maintain oxygenation or anoxia, respectively. The hippocampi are quickly desiccated and introduced in a chopper (Mclllwain) to cut them into slices of 350 microns thick. The hippocampus slices are immediately transferred to tubes containing the same solution without sacarose and are bubbled with carbogen for an hour to stabilise them. Six groups are evaluated in each individual experiment. These groups are distributed into four Atremorine concentrations (10 and 100 ng/ml and 1 and 10 µg/ml), in addition to the (basal) control and the OGD group (group of slices subjected to glucose and oxygen deprivation without treatment). The slices are pre-incubated for 30 minutes in a normal Krebs solution containing (in mM): NaCl 120, KCI 2, CaCl₂ 2, NaHCO₃ 26, MgSO₄ 1.19, KH₂PO₄ 1.18 and glucose 11. Oxygen and glucose deprivation is induced by bubbling nitrogen and replacing the glucose with 2-deoxyglucose. The viability of the slices was evaluated using the MTT method.

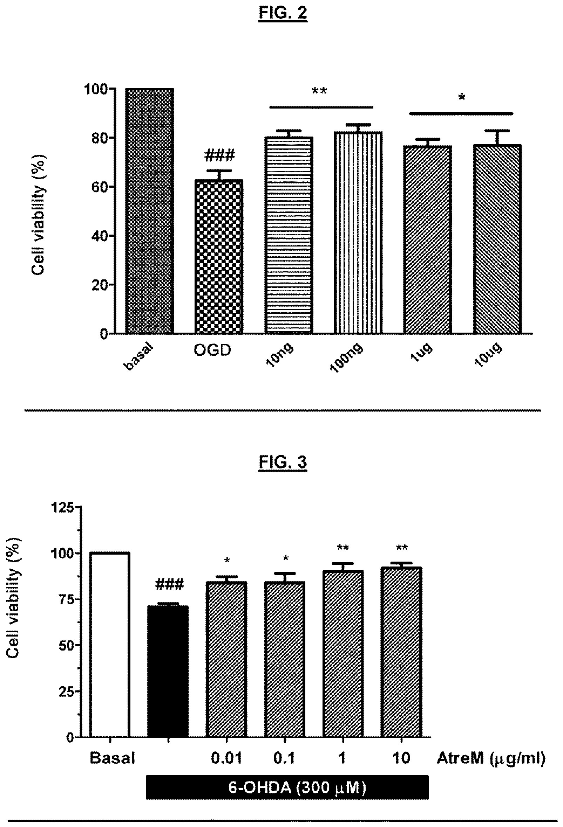

As can be observed in figure 2, the exposure of the hippocampus slices to OGD for 15 minutes followed by 2 hours of reoxygenation produced a cell death of approximately 40%. In these conditions, the incubation of Atremorine produced a significant protective effect at all the assayed concentrations, with maximum protection at 100 ng/ml (50%).

### 1.3 Evaluation of the protective effect of Atremorine in the 6-OHDA toxicity model on slices of rat striatum.

These experiments were conducted on Sprague-Dawley rats from a colony at the animal facility. The rats are anaesthetised using sodium barbital (69 mg/kg, i.p.). Next, they are decapitated and the brain removed and quickly immersed in a pre-cooled bicarbonate Krebs solution (with high magnesium and sacarose and low calcium content) and whose composition, in mM, is as follows: NaCl 120, KCI 2, CaCl₂ 0,5, NaHCO₃26, MgSO₄ 10, KH₂PO₄ 1.18, glucose 11 and sacarose 200. All the solutions are previously bubbled using 95% O₂/5% CO₂ (carbogen). The striatum is quickly desiccated and introduced in a chopper (Mclllwain) to cut them into slices of 350 microns thick. The striatum slices are immediately transferred to tubes containing the same solution without sacarose and are bubbled with carbogen for an hour to stabilise them. Six groups are evaluated in each individual experiment. These groups are distributed into four Atremorine concentrations (0.01, 0.1, 1 and 10 µg/ml), in addition to the (basal) control and the 300 µM 6-OHDA group. The slices are pre-incubated for 30 minutes in a normal Krebs solution containing (in mM): NaCl 120, KCI 2, CaCl₂ 2, NaHCO₃ 26, MgSO₄ 1.19, KH₂PO₄ 1.18 and glucose 11. Toxicity was induced by incubation with 300 µM 6-hydroxydopamine for 4 hours. The viability of the slices was evaluated by means of the MTT method.

As can be observed in figure 3, the incubation of the slices with 300 µM 6-OHAD for 4 hours produced 29% cell death. In these conditions, incubation of Atremorine produced a concentration-dependent protective effect, with maximum protection at 10 µg/ml (72%).

### EXAMPLE 2. In vivo studies.

The studies described below analyse the effect of Atremorine on the survival of dopaminergic nigrostriatal neurons (DA) in the 1-methyl-4-phenyl-1,2,3,6-tetrahydropyridine (MPTP) murine model of Parkinson's Disease. The Parkinsonian effects induced by the degeneration of MPTP of the nigrostratal DA neurons were studied by means of anti-tyrosin hydroxylase markers, microglial activation and neuronal death. Using immunohistochemical techniques, it was observed that treatment with Atremorine has a neuroprotective effect and prevents the degeneration of nigrostratal DA neurons by 46%, increasing the dopamine levels of the striatum, and the enhanced motor and learning functions. These results show that Atremorine has neuroprotective and anti-inflammatory effects against the degenerative effects of Parkinson's Disease, due to which Atremorine has therapeutical value in the treatment of the neurodegenerative aspects of Parkinson's Disease related to dementia and cognitive impairment in humans.

The objective was to study the effect of Atremorine as an anti-Parkinsonian agent in mice.
Types of animal models: wild laboratory mice (C57BL6/J 10 weeks)
   - Wild mice with chemically induced neurodegeneration (q/i)
   - Wild mice (control)
Neurodegeneration induced by: MPTP
   - 0.5 mg / mouse (weight: 25 mg/Kg) - 100 µl / mouse - i.p.
Treatments:
   - Atremorine (2 mg) - 250 µl / mouse
   - Atremorine (4 mg) - 500 µl / mouse
   - L-DOPA (Sinemet Plus: 2 mg) - 200 µl / mouse - i.p.
Experimental strategy:
   1. Behavioural analysis Rota-Rod OUCEM-86
      Coordination of movements
      Hypokinetic motor deficiency
   2. Processing and histopathological analysis of affected brain regions -Dysfunction and degeneration of DA neurons

**Table 1. Experimental table.**

| | **Experimental treatment** | **n** | **n mouse q/i** |
|---|---|---|---|
| **Group A** | **MPTP / 2 mg Atremorine in food** | **6** | **6** |
| **Group B** | **MPTP / 4 mg Atremorine in food** | **6** | **6** |
| **Group C** | **MPTP / 2 mg L-DOPA (anti-Parkinsonian treatment)** | **6** | **6** |
| **Group D** | **MPTP / 2 mg Atremorine / 2 mg L-DOPA)** | **6** | **6** |
| **Group E** | **MPTP (chemical neurodegeneration)** | **4** | **4** |
| **Group F** | **4 mg Atremorine in food (control)** | **4** | **4** |
| **Group G** | **Food (control)** | **4** | **4** |

### Histopathological and neurochemical characteristics of the animal model with MPTP.

A histopathological study of one of the individuals that developed Parkinsonian syndrome after being injected a drug containing MPTP showed a selective lesion of the dopaminergic cells of the SNpc; however, studies carried out on primates have demonstrated that the MPTP-induced lesion does not exclusively affect this neuronal population. In this respect, different groups have demonstrated that in adult macaques the administration of MPTP also induces degeneration of the NDAs of the ventral tegmental area (VTA) and of the *locus coeruleus,* where eosinophilic inclusion bodies similar to Lewy bodies have been described. The degeneration of the VTA, although to a lesser extent, is also evident in young primates, although the neuronal loss is always of greater intensity in the NDAs of the SNpc. From the neurochemical viewpoint, there is also a great similarity between MPTP-induced Parkinsonism and PD. The neurotoxic action of MPTP is characterised, like PD, by a reduction in the concentration of dopamine (DA) and its metabolites, 3,4-dihydroxyphenylacetic acid (DOPAC) and homovanillic acid (HVA), as well as a reduction in the activity of the hydroxylase tyrosine enzyme and an alteration in the density of dopaminergic receptors. As opposed to what happens in PD, where the reduction in DA is more intense in the putamen than in the caudate nucleus, the primates chronically exposed to the action of MPTP showed a homogeneous and non-selective reduction in striatal DA content, being of the same intensity in both striatal structures. Other brain regions with dopaminergic innervation such as the nucleus accumbens, the hippocampus, the amygdale and the cerebral cortex, are also affected by MPTP and the reduction in DA content is similar to that described in the striatum. Contrarily, DA levels are not altered in structures such as the lateral and medial pale globe, which receives direct projections from the SNpc. Additionally, the concentrations of noradrenaline and serotonin in striatum are not modified by the acute administration of MPTP, although they are significantly reduced in the striatum and cortex in the chronically treated animals.

### Atremorine protects against MPTP-induced dopaminergic neurodegeneration.

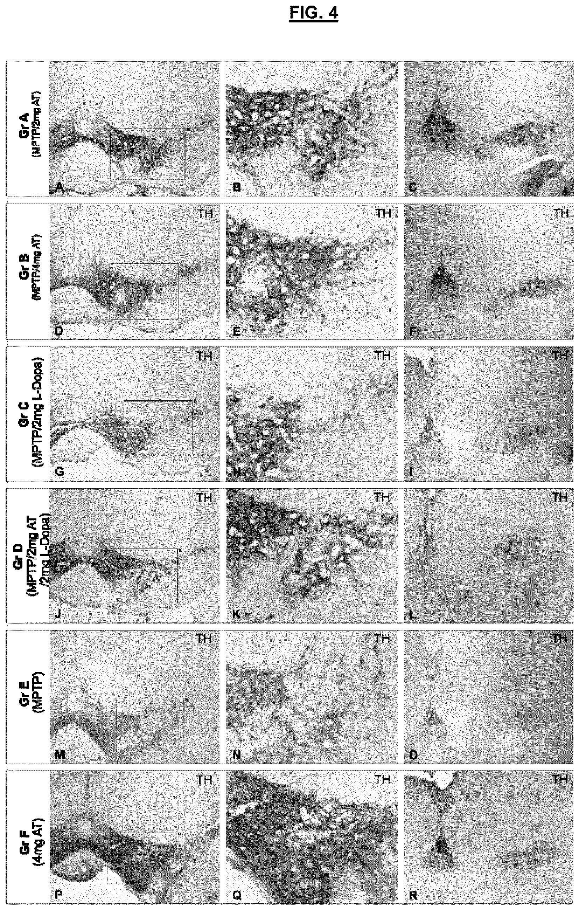

The mice were treated with Atremorine (treatment) or L-DOPA (Sinemet Plus, control) through diet. After seven days of treatment the animals were processed to quantify the dopaminergic cell bodies in the SNpc and their dopaminergic fibrillar projections in the striatum by means of immunostaining of hydroxylase tyrosine (HT). The chemical poisoning induced by MPTP resulted approximately in a 71% loss of HT-positive neurons of the SNpc (group E, fig. 4) compared to the untreated healthy normal controls (group G). However, in MPTP-induced mouse models treated with Atremorine (groups A and B) a significant neuroprotective effect was observed, resulting from a lower reduction in the density of HT-positive neurons in the SNpc (fig. 4A-F). The dosis of 2 mg of Atremorine was slightly more efficient in the protection of the HT-positive neurons and fibres against MPTP toxicity (fig. 4A-C), 25% loss of HT-positive neurons of the SNpc. Additionally, the isolated and combined effect of the drug Sinemet Plus was also quantified (2 mg L-DOPA (group C) / L-DOPA+Atremorine (group D)). The results observed indicate that the combined effect of Atremorine and L-DOPA (Sinemet Plus) increase the neuroprotective effect (fig. 4G-L) and, consequently, results in a significant reduction in neuronal damage, 44% loss of HT-positive neurons of the SNpc with direct effect on nigrostriatal neurodenegeration, thereby mitigating the pathological effect on the central nervous system observed in the MPTP controls (fig. 4M-O). The neuroprotective effect of Atremorine in dopaminergic neuronal populations was also observed in group F (fig. 4P-R), wherein said population expresses HT at slightly higher levels than the normal untreated control (group G).

### Atremorine inhibits MPTP-induced microglial activation in the substantia nigra.

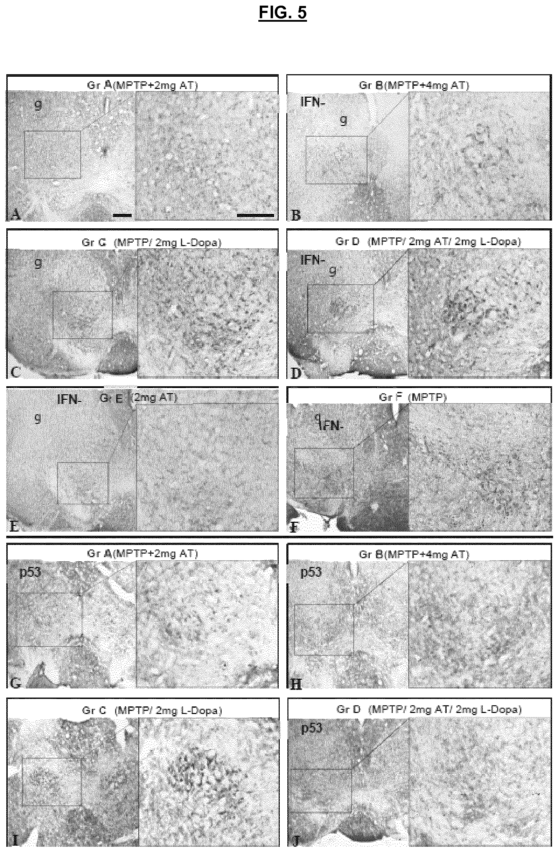

It has been demonstrated that there is activation of astrocytes in the *substantia nigra* (SN) of Parkinson's patients, as well as in mice treated with MPTP, triggering a neuro-inflammatory process. Therefore, this study also examines whether or not Atremorine inhibits the microglial activation induced by MPTP in the SN, in order to improve neuronal survival. To this end, immunohistochemical assays were conducted using specific antibodies against microglia (IFN-y) and in all the mice groups undergoing treatment. Treatment with Atremorine after the MPTP injection dramatically reduced the density of activated microglia in mice treated with MPTP (fig. 5A-B). In accordance with prior studies, inactivated microglia and astrocytes were transformed into cells with enlarged bodies and thick dendrites in mice treated with MPTP (fig. 5C-F). As a control, Atremorine on its own did not have adverse effects (fig. 5E).

### Atremorine inhibits MPTP-induced cell death in the substantia nigra.

An essential characteristic in the neuronal impairment of the SN in Parkinson's Disease is cell degeneration and also in mice treated with MPTP, triggering apoptotic and necrosis marker activation processes. Therefore, this study also examines whether or not Atremorine inhibits MPTP-induced cell death in the SN to protect neurochemical stability. To this end, immunohistochemical assays were conducted using specific antibodies against apoptosis (p53), in all the mice groups undergoing treatment. Treatment with Atremorine after the MPTP injection dramatically reduced the number of neuronal cell deaths in mice (fig. 5G-H). Additionally, it was observed that the effects of MPTP-induced cell death (fig. 5I) are reduced with the combination of Atremorine and L-DOPA (fig. 5J).

### Atremorine enhances motor functions in mice with MPTP-induced neurodegeneration.

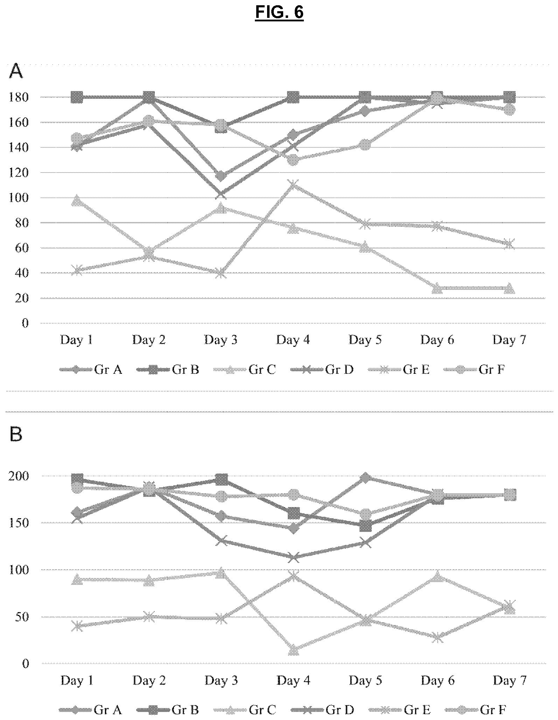

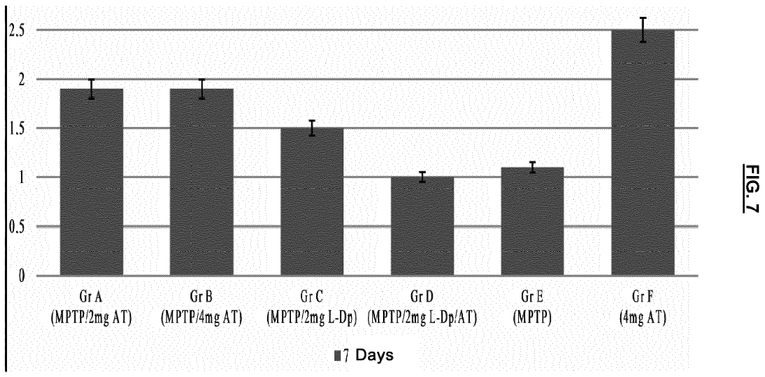

The therapeutic objective of neuroprotection is to reduce the functional impairment of the nervous system. Therefore, behavioural assays were conducted in order to examine whether or not Atremorine not only protects against structural and neurotransmission damage but also against the functional deficiencies caused by MPTP, which affects the locomotion and movement coordination index. The results of said assays on the rotating rod performance test showed that groups A, B, D and F maintained a motor activity similar to that of the normal controls, whereas the mice of groups C and E lost motor agility, coordination of movements and have a marked decrease in muscle tone and balance, both before and after each treatment (fig. 6). MPTP-induced hypolocomotion (group E, fig. 6) was also observed to a large extent in the mice of group C. As regards the best performance at motor level, the potentiating effect of Atremorine should be noted, since group B (before each treatment) and group F (after each treatment) demonstrated a significantly high level of motor activity and coordination at 16 rpm (p<0.0001). Additionally, learning tests (OUCEM) were conducted to analyse the neurodegenerative effect of MPTP on the different groups of mice. The results obtained are shown in figure 7 and demonstrate that the neuroprotective effect of Atremorine avoids dopaminergic cell degeneration and the ensuing learning deficiency.

### Example 3. Clinical assays.

A group of 92 patients (47 women, 45 men) with Parkinson's Disease and other Parkinsonian syndromes (toxic, traumatic, degenerative, ischemic) was selected (see characteristics of the sample in Table 2). Of the total, 78 patients had never received prior treatment and the rest were undergoing treatment with Sinemet at different doses. The patients underwent a full medical examination: (i) anamnesis and clinical evaluation; (ii) psychometry; (iii) blood test (see Table 2); (iv) neuroimaging tests (brain MRI); (v) brain cartography; (vi) genetic profile (APOE); and (vii) pharmacogenetic profile (CYP2D6). After informed consent, the patients were administered an oral dose of 5 g of Atremorine and were subject to a basal analysis (prior to ingestion) and 30 minutes after the ingestion of Atremorine in a yoghurt. The analytical parameters studied were: dopamine (DA), noradrenalin (NA), prolactin (PRL), cortisol (Cort) and growth hormone (GH). Untreated patients and patients undergoing chronic treatment with anti-Parkinsonian drugs were differentiated in the studies. The pharmacogenetic studies always relate to patients receiving Atremorine for the first time and who have never previously been treated with Sinemet or other anti-Parkinsonian treatments.

The APOE genotypes are distributed with the following frequencies: APOE-2/3, 13.80%; APOE-3/3, 63.10%; and APOE-3/4, 23.10% (no patient presented APOE-2/2, APOE-2/4 or APOE-4/4 genotypes).

The CYP2D6 phenotypes were distributed as follows: (i) CYP2D6-EM extensive metabolisers, 53.62%; (ii) CYP2D6-IM intermediate metabolisers, 39.13%; (iii) CYP2D6-PM poor metabolisers, 4.35%; and (iv) CYP2D6-UM ultra-rapid metabolisers, 2.90%.

### Effect of E-PodoFavalin-15999 on serum dopamine levels.

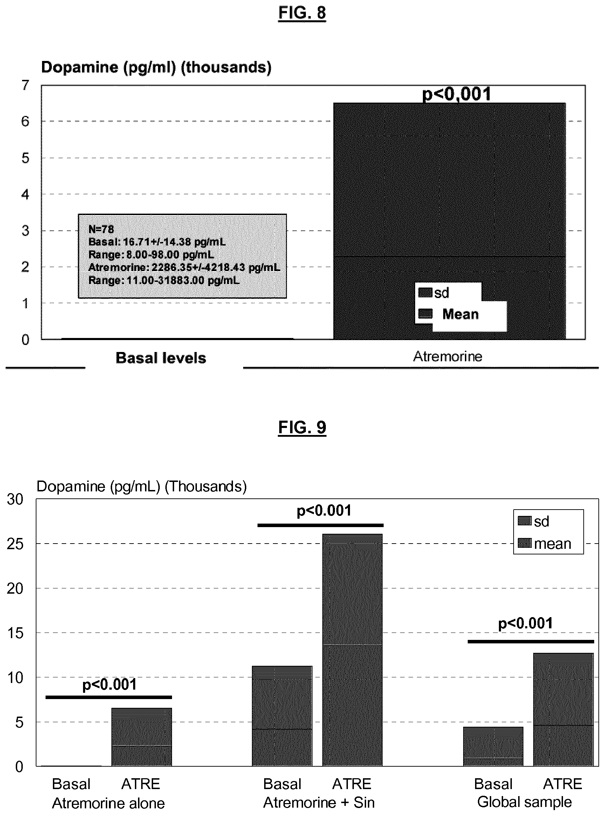

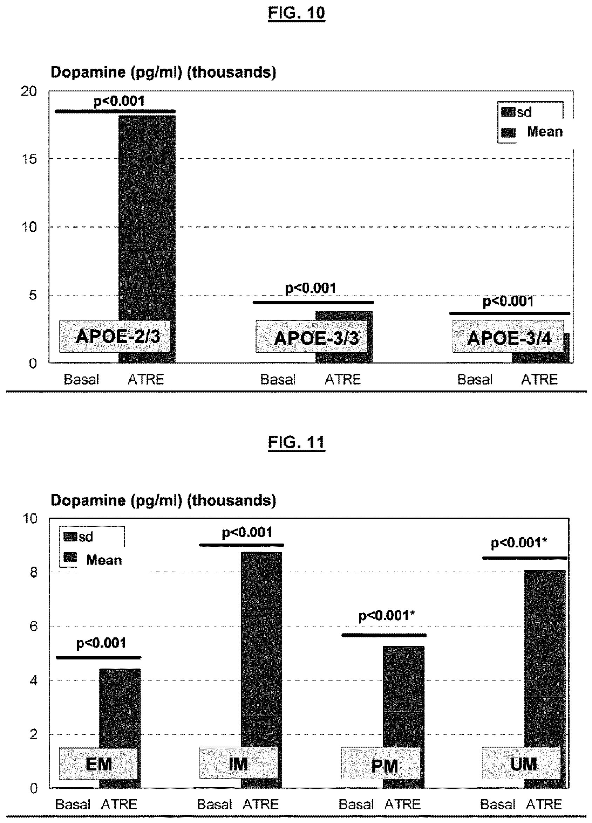

The oral administration of 5 g of Atremorine induces a spectacular serum dopamine response in 98.92% of patients (fig. 8). This response is similar in patients who have never previously been treated with anti-Parkinsonian medication (fig. 8) and in those who are receiving Sinemet (fig. 9). The co-administration of Atremorine and Sinemet potentiates the effect of Sinemet. The serum dopaminergic response range in previously untreated patients ranges between 11 and 31883 pg/mL and in patients treated with Sinemet ranges between 1326 and 40603 pg/mL (fig. 8 and 9). The response of dopamine to the administration of Atremorine has a certain pharmacogenetic component (fig. 10 and 11): although all patients respond clearly, the patients who best responded are APOE-2/3, followed by APOE-3/3 and APOE-3/4 (fig. 10). Similarly, CYP2D6-IM (intermediate metabolisers) showed a stronger response than CYP2D6-EM (extensive metabolisers) (fig. 11). The response of PMs and UMs is not assessable due to the scarce number of patients with this pharmacogenetic condition.

**Table 2. Basal analysis of the sample of Parkinson's patients.**

| **Parameters** | **Value (m ± ds)** |
|---|---|
| N (Women: 47; Men: 45) | 92 |
| Age (years) | 60.60±15.55 |
| Range | 21-89 |
| Systolic Blood Pressure (mm Hg) | 137.04±24.19 |
| Diastolic Blood Pressure (mm Hg) | 78.16±11.23 |
| Pulse (Ipm) | 73.11±12.38 |
| Weight (kg) | 73.11±12.38 |
| Height (m) | 1.62±0.09 |
| BMI (kg/m²) | 26.64±4.32 |
| Glucose (mg/dL) | 99.98±17.35 |
| Total cholesterol (mg/dL) | 200.72±38.55 |
| HDL cholesterol (mg/dL) | 57.58±12.9 |
| LDL cholesterol (mg/dL) | 120.50±32.06 |
| Triglycerides (mg/dL) | 111.29±60.39 |
| Urea (mg/dL) | 43.88±12.91 |
| Creatinin (mg/dL) | 0.91±0.20 |
| Uric acid (mg/dL) | 4.72±1.38 |
| Total proteins (g/dL) | 6.91±0.50 |
| Calcium (mg/dL) | 9.66±0.49 |
| GOT/ASAT (IU/L) | 20.97±7.41 |
| GPT/ALAT (IU/L) | 23.54±16.91 |
| GGT (IUIL) | 29.26±34.51 |
| Na⁺ (mEq/L) | 140.33±4.21 |
| K⁺ (mEq/L) | 4.27±0.27 |
| Cl⁻ (mEq/L) | 102.25±6.53 |
| Fe²⁺ (µg/dL) | 82.02±32.52 |
| Ferritin (ng/mL) | 159.84±147.15 |
| Folate (ng/mL) | 15.99±6.65 |
| Vitamin B₁₂(pg/mL) | 738.02±419.50 |
| TSH (µlU/mL) | 1.66±1.35 |
| T4 (ng/mL) | 0.89±0.17 |
| RBC (x10⁶/µL) | 4.59±0.46 |
| HCT (%) | 42.31±3.88 |
| Hb (g/dL) | 14.12±1.57 |
| VCM (fL) | 92.19±4.13 |
| HCM (pg) | 31.00±1.49 |
| CHCM (g/dL) | 33.63±0.76 |
| ADE (RDW) (%) | 12.92±1.12 |
| WBC (x10³/µL) | 6.75±1.57 |
| %Neu | 42.47±26.01 |
| %Lin | 31.77±8.54 |
| %Mon | 7.26±1.57 |
| %Eos | 2.73±1.30 |
| %Bas | 0.71±0.48 |
| PLT (x10³/µL) | 213.33±58.09 |
| VPM (fL) | 8.75±0.84 |
| VSG | 16.95±9.96 |

### Effect of Atremorine on serum noradrenaline levels in Parkinson's patients.

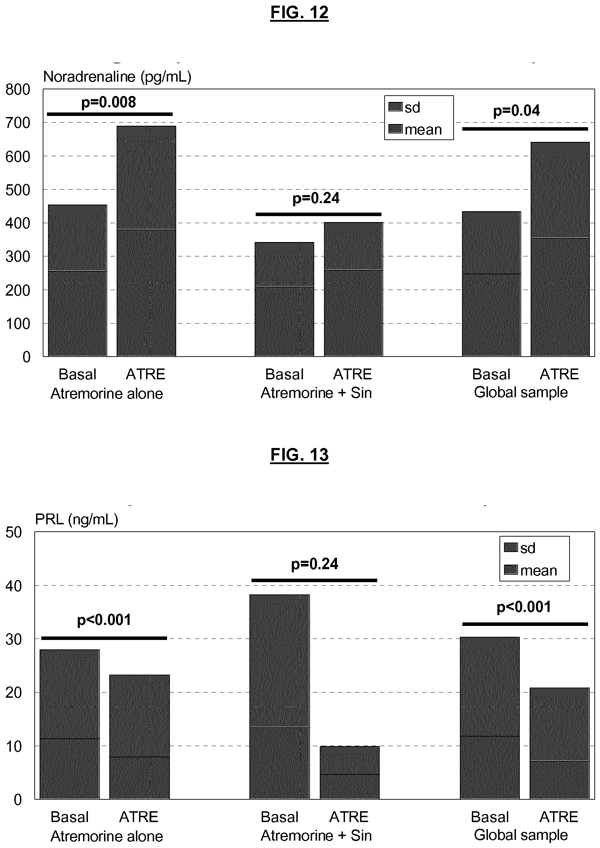

Atremorine produces a significant increase in serum noradrenaline levels in Parkinson's patients (fig. 12), particularly in previously untreated patients. In those who follow continued treatment with Sinemet (fig. 12), the response of Noradrenaline to Atremorine is annulled. This response is similar in patients with APOE-2/3 and APOE-3/3 genotype and is slightly lower in APOE-3/4 patients. This response does not seem to be affected by the CYP2D6 condition, although CYP2D6-EM and CYP2D6-IM patients seem to respond better than CYP2D6-PM and CYP2D6-UM patients.

### Effect of Atremorine on serum prolactin (PRL) levels in Parkinson's patients.

The oral administration of Atremorine induces a significant reduction in serum PRL levels in previously untreated patients (fig. 13). This response is also significant in patients pretreated with anti-Parkinsonian drugs, but more variable (fig. 13). APOE-3/3 patients show the highest basal PRL levels and the most potent response to Atremorine, followed in potency by the response observed in APOE-3/4 patients and minimum response of APOE-2/3 patients. The basal PRL levels show a certain dependence on the CYP2D6 profile. CYP2D6-EM patients showed the highest basal levels and the most potent response to Atremorine, followed by CYP2D6-IM, CYP2D6-PM and CYP2D6-UM patients.

### Effect of Atremorine on serum cortisol levels in Parkinson's patients.

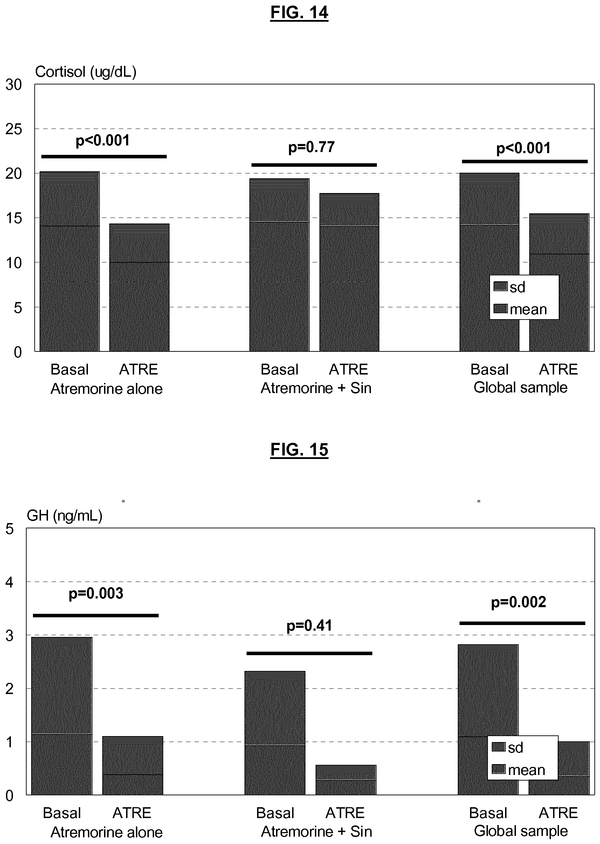

The oral administration of Atremorine caused a reduction in cortisol in previously untreated patients (fig. 14). This response disappears in patients undergoing chronic treatment with conventional anti-Parkinsonian drugs (fig. 14). The decrease in cortisol levels in response to Atremorine is similar in APOE-2/3, APOE-3/3 and APOE-3/4 patients, with a potent response in APOE-3/3 patients. The response is also similar in CYP2D6-EM and CYP2D6-IM patients. The trend is identical in CYP2D6-PM patients and disappears in CYP2D6-UM patients, but the data in PMs and UMs must be considered with reservations due to the low number of cases in both groups.

### Effect of Atremorine on serum growth hormone (GH) levels in Parkinson's patients.

The oral administration of Atremorine induces a significant decrease in serum GH levels in previously untreated Parkinson's patients (fig. 15). This response is less significant in patients pretreated with conventional anti-Parkinsonian drugs (fig. 15). The basal GH levels are higher in APOE-2/3 patients, followed by APOE-3/3 and APOE-3/4 patients. The response to Atremorine is significant in APOE-2/3 and APOE-3/3 patients and insignificant in APOE-3/4 patients. In relation to the CYP2D6 genotype, the response of GH to Atremorine is significant in CYP2D6-EM patients, with an identical trend in CYP2D6-IM, CYP2D6-PM and CYP2D6-UM. The basal GH levels are maximum in CYP2D6-PM.

## Claims

1. A process for obtaining a bioactive extract from the *Vicia faba* pod that comprises the following stages:
a. harvesting the fruit of a *Vicia faba* crop during the growth phase,
b. removing the beans from the fruit harvested in stage (a) and processing the pods by means of a treatment that comprises the steps of washing the pods, rejecting the damaged and/or oxidised pods and washing and quick-drying the selected pods,
c. preserving the product obtained in stage (b) at a temperature between 0ºC and 5ºC for 24-48 hours,
d. deep-freezing the product obtained after stage (c) at between - 50ºC and -30ºC for 1-3 hours,
e. storing the product obtained after stage (d) at a temperature below -18ºC for less than three months,
f. freeze-drying the product obtained after stage (e) for 3-5 days, and
g. standardising the L-DOPA content of the product obtained after stage (f) such that it has a final L-DOPA content of at least 20 mg/g.

2. The process according to claim 1, wherein in step (a) the fruit is harvested two to three weeks before the crop enters the maturation phase.

3. The process according to any of claims 1 or 2, wherein the *Vicia faba* crop is an organic crop.

4. The process according to any of claims 1 to 3, wherein the *Vicia faba* crop is of the variety *Vicia faba* L. (sp. Var. MM).

5. The process according to any of claims 1 to 4, which also comprises a stage (h) of homogenisation of the product obtained after stage (g), which comprises standardizing the texture and appearance of the final product by means of a neutral physical treatment.

6. The process according to any of claims 1 to 5, which also comprises a stage (i) of formulation in which between 0.020 g and 0.040 g of D-Alpha-Tocoferol are added.

7. A bioactive extract obtained by means of the process according to any of claims 1 to 6, preferably wherein said extract is in powdered form.

8. The bioactive extract according to claim 7, wherein said extract comprises an amount of L-DOPA of at least 20 mg/g and amounts of vicine and convicine of less than 2 mg/g, preferably less than 1 mg/g.

9. A pharmaceutical composition that comprises the bioactive extract according to any of claims 7 or 8, preferably wherein the bioactive extract is found in an amount of between 3 g and 7 g, more preferably 5 g.

10. The pharmaceutical composition according to claim 9, which also comprises a pharmaceutically acceptable vehicle and/or other active ingredient, preferably wherein said active ingredient is an anti-Parkinsonian drug, more preferably L-DOPA.

11. The pharmaceutical composition according to any of claims 9 or 10, wherein said composition is formulated for the oral administration thereof.

12. The pharmaceutical composition according to any of claims 9 to 11, wherein said composition is included in a capsule, bag or bottle.

13. The bioactive extract according to any of claims 7 or 8, or the pharmaceutical composition according to any of claims 9 to 12, for use as a medicament.

14. The bioactive extract according to any of claims 7 or 8, or the pharmaceutical composition according to any of claims 9 to 12, for use in the treatment and/or prevention of neurodegenerative diseases, preferably Parkinson's Disease or other Parkinsonian syndromes.

15. The bioactive extract or pharmaceutical composition for use according to claim 14, wherein the disease is Parkinson's Disease, preferably Parkinson's Disease in patients with APOE-2/3 genotype and/or CYP2D6-IM phenotype.

## Patentansprüche

1. Verfahren zum Erhalten eines bioaktiven Extrakts aus der *Vicia faba-*Hülse, das folgende Stufen umfasst:
a. Ernten der Frucht einer *Vicia* faba-Kultur während der Wachstumsphase,
b. Entfernen der Bohnen aus der in Stufe (a) geernteten Frucht und Verarbeiten der Hülsen mittels einer Behandlung, die die Schritte des Waschens der Hülsen, des Zurückweisens der beschädigten und/oder oxidierten Hülsen und des Waschens und schnellen Trocknens der ausgewählten Hülsen umfasst,
c. Konservieren des in Stufe (b) erhaltenen Produkts bei einer Temperatur zwischen 0 ºC und 5 ºC für 24-48 Stunden,
d. Tiefkühlen des nach Stufe (c) erhaltenen Produkts bei Temperaturen zwischen -50 ºC und -30 ºC für 1-3 Stunden,
e. Lagerung des nach der Stufe (d) erhaltenen Produkts bei einer Temperatur unter -18 ºC für weniger als drei Monate,
f. Gefriertrocknen des nach Stufe (e) erhaltenen Produkts für 3-5 Tage, und
g. Standardisierung des L-DOPA-Gehalts des nach Stufe (f) erhaltenen Produkts, so dass es einen endgültigen L-DOPA-Gehalt von mindestens 20 mg/g aufweist.

2. Verfahren nach Anspruch 1, wobei in Schritt (a) die Frucht zwei bis drei Wochen vor Eintritt der Kultur in die Reifephase geerntet wird.

3. Verfahren nach einem der Ansprüche 1 oder 2, wobei die *Vicia faba-*Kultur eine organische Kultur ist.

4. Verfahren nach einem der Ansprüche 1 bis 3, wobei die *Vicia* faba-Kultur von der Sorte *Vicia faba* L. (sp. Var. MM) ist.

5. Verfahren nach einem der Ansprüche 1 bis 4, das auch eine Stufe (h) der Homogenisierung des nach Stufe (g) erhaltenen Produkts umfasst, umfassend die Standardisierung der Textur und des Aussehens des Endprodukts mittels einer neutralen physikalischen Behandlung.

6. Verfahren nach einem der Ansprüche 1 bis 5, das auch eine Stufe (i) der Formulierung umfasst, in der zwischen 0,020 g und 0,040 g D-Alpha-Tocoferol zugegeben werden.

7. Bioaktiver Extrakt, erhalten durch das Verfahren nach einem der Ansprüche 1 bis 6, vorzugsweise wobei der Extrakt in Pulverform vorliegt.

8. Bioaktiver Extrakt nach Anspruch 7, wobei der Extrakt eine Menge an L-DOPA von mindestens 20 mg/g und Mengen an Vicin und Convicin von weniger als 2 mg/g, vorzugsweise weniger als 1 mg/g umfasst.

9. Pharmazeutische Zusammensetzung, die den bioaktiven Extrakt nach einem der Ansprüche 7 oder 8 umfasst, wobei vorzugsweise der bioaktive Extrakt in einer Menge zwischen 3 g und 7 g, insbesondere bevorzugt 5 g, vorhanden ist.

10. Pharmazeutische Zusammensetzung nach Anspruch 9, die auch einen pharmazeutisch verträglichen Träger und/oder einen anderen Inhaltsstoff umfasst, wobei vorzugsweise der Inhaltsstoff ein Parkinson-Mittel, insbesondere bevorzugt L-DOPA, ist.

11. Pharmazeutische Zusammensetzung nach einem der Ansprüche 9 oder 10, wobei die Zusammensetzung für ihre orale Verabreichung formuliert ist.

12. Pharmazeutische Zusammensetzung nach einem der Ansprüche 9 bis 11, wobei die Zusammensetzung in einer Kapsel, einem Beutel oder einer Flasche enthalten ist.

13. Bioaktiver Extrakt nach einem der Ansprüche 7 oder 8, oder pharmazeutische Zusammensetzung nach einem der Ansprüche 9 bis 12 zur Verwendung als Medikament.

14. Bioaktiver Extrakt nach einem der Ansprüche 7 oder 8, oder pharmazeutische Zusammensetzung nach einem der Ansprüche 9 bis 12 zur Verwendung bei der Behandlung und/oder Vorbeugung von neurodegenerativen Erkrankungen, vorzugsweise Morbus Parkinson oder anderen parkinsonschen Syndromen.

15. Bioaktiver Extrakt oder pharmazeutische Zusammensetzung zur Verwendung nach Anspruch 14, wobei die Krankheit Morbus Parkinson ist, vorzugsweise Morbus Parkinson bei Patienten mit APOE-2/3-Genotyp und/oder CYP2D6-IM-Phänotyp.

## Revendications

1. Procédé pour l'obtention d'un extrait bioactif de la cosse de *Vicia faba* qui comprend les étapes suivantes :
a. cueillir le fruit d'une culture de *Vicia faba* pendant la phase de croissance,
b. retirer les fèves du fruit cueilli à l'étape (a) et traiter les cosses au moyen d'un traitement qui comprend les étapes consistant à laver les cosses, rejeter les cosses endommagées et/ou oxydées et laver et sécher rapidement les cosses choisies,
c. conserver le produit obtenu à l'étape (b) à une température entre 0 ºC et 5 ºC pendant 24-48 heures,
d. surgeler le produit obtenu après l'étape (c) entre - 50 ºC et -30 ºC pendant 1-3 heures,
e. stocker le produit obtenu après l'étape (d) à une température en-dessous de -18 ºC pendant moins de trois mois,
f. lyophiliser le produit obtenu après l'étape (e) pendant 3-5 jours, et
g. uniformiser la teneur en L-DOPA du produit obtenu après l'étape (f) de sorte qu'il ait une teneur finale en L-DOPA d'au moins 20 mg/g.

2. Procédé selon la revendication 1, dans lequel à l'étape (a) le fruit est cueilli deux à trois semaines avant que la culture n'entre dans la phase de maturation.

3. Procédé selon l'une quelconque des revendications 1 ou 2, dans lequel la culture *Vicia faba* est une culture biologique.

4. Procédé selon l'une quelconque des revendications 1 à 3, dans lequel la culture *Vicia faba* est de la variété *Vicia faba* L. (sp. Var. MM).

5. Procédé selon l'une quelconque des revendications 1 à 4, qui comprend également une étape (h) d'homogénéisation du produit obtenu après l'étape (g), qui comprend l'uniformisation de la texture et de l'apparence du produit final au moyen d'un traitement physique neutre.

6. Procédé selon l'une quelconque des revendications 1 à 5, qui comprend également une étape (i) de formulation dans laquelle entre 0,020 et 0,040 g de D-Alpha-Tocophérol sont ajoutés.

7. Extrait bioactif obtenu au moyen du procédé selon l'une quelconque des revendications 1 à 6, de préférence dans lequel ledit extrait est sous forme de poudre.

8. Extrait bioactif selon la revendication 7, dans lequel ledit extrait comprend une quantité de L-DOPA d'au moins 20 mg/g et des quantités de vicine et de convicine inférieures à 2 mg/g, de préférence inférieures à 1 mg/g.

9. Composition pharmaceutique qui comprend l'extrait bioactif selon l'une quelconque des revendications 7 ou 8, de préférence dans laquelle l'extrait bioactif est présent dans une quantité comprise entre 3 g et 7 g, plus préférablement 5 g.

10. Composition pharmaceutique selon la revendication 9, qui comprend également un véhicule pharmaceutiquement acceptable et/ou autre ingrédient actif, de préférence dans laquelle ledit ingrédient actif est un médicament antiparkinsonien, plus préférablement L-DOPA.

11. Composition pharmaceutique selon l'une quelconque des revendications 9 ou 10, dans laquelle ladite composition est formulée pour l'administration orale de celle-ci.

12. Composition pharmaceutique selon l'une quelconque des revendications 9 à 11, dans laquelle ladite composition est inclue dans une capsule, une poche ou bouteille.

13. Extrait bioactif selon l'une quelconque des revendications 7 ou 8, ou composition pharmaceutique selon l'une quelconque des revendications 9 à 12, pour son utilisation comme médicament.

14. Extrait bioactif selon l'une quelconque des revendications 7 ou 8, ou composition pharmaceutique selon l'une quelconque des revendications 9 à 12, pour son utilisation dans le traitement et/ou la prévention de maladies neurodégénératives, de préférence la maladie de Parkinson ou autres syndromes Parkinsoniens.

15. Extrait bioactif ou composition pharmaceutique pour son utilisation selon la revendication 14, dans lequel la maladie est la maladie de Parkinson, de préférence la maladie de Parkinson chez des patients atteints du génotype APOE-2/3 et/ou phénotype CYP2D6-IM.
